# EUROPEAN PATENT SPECIFICATION

(11) **EP 1 820 889 B1**
(45) Date of publication and mention of the grant of the patent: **18.03.2009**
(21) Application number: 07250342.8
(22) Date of filing: 27.01.2007
(51) Int. Cl.: D02G 3/04, D02G 3/22, D02G 3/44

(54) **Yarns useful for constructing graft materials**
Garne zur Herstellung von Transplantationsmaterialien
Fils utiles à la construction de matériaux de greffe

(30) Priority: 17.02.2006 US 356677
(43) Date of publication of application: 22.08.2007
(73) Proprietor: Cordis Corporation, Miami Lakes, FL 33014 (US)
(72) Inventor: Dwyer, Clifford J., Weston, Florida 33331 (US)
(74) Representative: Belcher, Simon James

(56) References cited:
- EP-A1- 0 445 872
- EP-A1- 0 519 359
- EP-B1- 1 126 055
- DE-A1- 1 760 229
- US-A- 3 589 956
- US-A- 4 610 925
- US-A- 4 765 812
- US-A- 5 454 846

## Description

The present invention relates to fibers and yarns useful as graft materials in vascular grafts or other graft devices. Particularly, the present invention relates to a composite yam, a co-extruded filament, and a reinforced fiber graft material. The present invention also relates to a process for assembling a graft device without using suture knots.

Graft devices have been widely used to replace malfunctioning biological structures or treat diseases associated therewith. For example, various vascular grafts are now Food and Drug Administration (FDA) approved and commercially available for treating a wide range of vascular diseases. A vascular graft typically comprises one or more stent segments, a graft material, and suture knots which are tied in such a way to affix the graft material to an outside portion of the one or more stent segments. The stent segment is either an expandable wire mesh or hollow perforated tube. The graft material is formed by fibers or yarns of biocompatible materials through a weaving, knitting, or braiding process.

One major challenge for developing a graft device, particularly, a vascular graft, is the lack of appropriate graft materials. The biostability and biocompatibility of the graft materials are critical for the use of graft devices. Since vascular grafts are intended for prolonged or permanent use and directly interface with body tissue, body fluids, and various biological molecules, the graft materials thereof must meet stringent biological and physical requirements.

When placed within the body in vessels, due to pulsatile blood pressure, a vascular graft, particularly, the graft material, is subject to high hydrodynamic forces and relative motion or rubbing between the stent segments and the graft material. Essentially, these forces and relative motion tend to wear the graft material at the points where it is connected to the stent segments. Over time, the graft material may develop microleaks which significantly undermine the performance of the vascular graft. Therefore, the graft material is required to be wear-resistant and highly durable. Furthermore, a vascular graft needs to conform to the anatomy of the patient's body without inducing detrimental stress. Thus, the graft material is required to be flexible and lubricious.

To achieve desirable flexibility and durability, prior art graft materials utilize yarns possessing different properties in blend. For example, a yarn of a flexible material and a yarn of a wear-resistant material may be used in combination to form a graft material. However, to satisfy the desired flexibility and durability, graft materials formed by blending different yarns are often too bulky. Since vascular grafts are mainly utilized to establish a fluid flow path from one section of a blood vessel to another section of the same or different blood vessel, it is preferred that a vascular graft has a low profile, i.e., small size. Lower profile also improves the maneuverability of a vascular graft. The size of a vascular graft can be reduced by employing thin-walled graft materials and/or decreasing the number of suture knots. However, the durability of thin-walled graft materials formed by conventional fibers or yarns is substantially less than that of graft materials having standard thickness. Furthermore, suture knots are important for securing the connection and minimizing the relative motion between the stent segments and the graft material. In a conventional vascular graft, almost each strut of each stent segment is secured to the graft material by suture knots.

Therefore, there remains a need for a fiber or yarn that can form flexible, wear-resistant, highly durable, and thin-walled graft materials.

Accordingly, the present invention provides a composite yarn as defined in appended claim 1 for construction of graft materials comprising at least one wear-resistant polymeric fiber and at least one flexible polymeric fiber. In the inventive composite yarn, the total number of the at least one wear-resistant polymeric fiber and the at least one flexible polymeric fiber ranges from about 5 to about 150, and the ratio of the at least one wear-resistant polymeric fiber to the at least one flexible polymeric fiber by number is about 1:4 to about 4:1. Preferably, the total number of the at least one wear-resistant polymeric fiber and the at least one flexible polymeric fiber ranges from about 10 to about 50.

The present invention can be used in a reinforced fiber graft material comprising wear-resistant beads and weaves of flexible polymeric fibers, wherein the wear-resistant beads are attached to the flexible polymeric fibers.

Embodiments of the invention will now be described by way of example with reference to the accompanying drawings, in which:

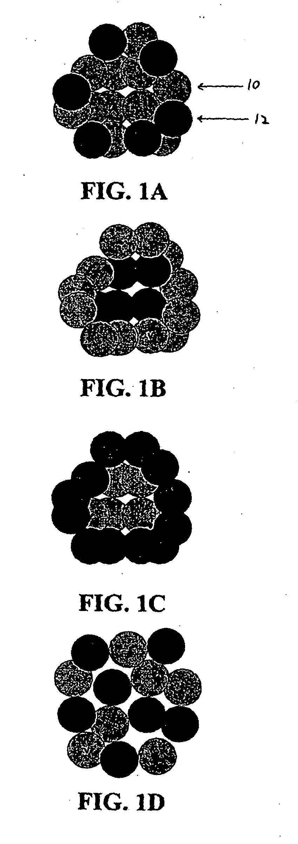

FIGS. 1A to 1D are pictorial cross-section illustrations of four embodiments of the inventive composite yam.

The present invention provides a composite yarn for construction of graft materials. The composite yarn comprises at least two types of fibers, i.e., at least one wear-resistant polymeric fiber and at least one flexible polymeric fiber. The total number of the at least one wear-resistant polymeric fiber and the at least one flexible polymeric fiber ranges from about 5 to about 150. Preferably, the total number of the at least one wear-resistant polymeric fiber and the at least one flexible polymeric fiber ranges from about 10 to about 50. The ratio of the at least one wear-resistant polymeric fiber to the at least one flexible polymeric fiber by number is about 1:4 to about 4:1. The term "yarn" as used herein denotes a long continuous length of interlocked fibers. The polymeric fiber of the present invention may be a monofilament or a co-extruded filament. By "monofilament", it is meant a single strand of fiber consisting of one polymeric material. By "co-extruded filament", it is meant a single strand of fiber formed by extruding at least two polymeric materials together. Preferably, the inventive composite yarn has a denier ranging from about 15 to about 500, and a tensile strength ranging from about 1.5 to about 3.5 Gpa. The term "denier" as used herein is defined as the mass in grams per 9000 meters.

The at least one wear-resistant polymeric fiber may be a fiber of any polymer that is stiff and wear-resistant. By "wear-resistant", it is meant being capable of withstanding the force or the effect of wear, including adhesive wear, abrasive wear, corrosive wear, and surface fatigue. Polymers suitable for the wear-resistant polymeric fiber of the present invention include, but are not limited to: polyolefin, polyester, poly(ether amide), poly(ether ester), poly(ether urethane), poly(ester urethane), poly(ethylene-styrene/butylene-styrene), and other block copolymers. Preferably, the wear-resistant polymeric fiber is a fiber of ultra high molecular weight polyethylene and ultra high molecular weight polypropylene. As used herein, the terms "ultra high molecular weight polyethylene" and "ultra high molecular weight polypropylene" denote a polyethylene having between six and twelve million ethylene units per molecule and a polypropylene having between six and twelve million propylene units per molecule, respectively. Ultra high molecular weight polyethylene is also known as UHMWPE or Dyneema^{®}.

The at least one flexible polymeric fiber may be a fiber of any polymer that is flexible and lubricous. Polymers suitable for the flexible polymeric fiber of the present invention include, but are not limited to: polyamide, polyester, polyolefin, and fluorinated polymer. Examples of the polymers suitable for the flexible polymeric fiber include, but are not limited to: nylon 6, nylon 66, nylon 11, nylon 12, polyethylene terphthalate, polybutylene terephthalate, low density polypropylene, low density polyethylene, and poly(vinylidene fluoride). Preferably, the flexible polymeric fiber is a fiber of polyethylene terphthalate, or polybutylene terephthalate. Polyethylene terphthalate is also known as Dacron^{®}. The terms "low density polypropylene" and "low density polyethylene" as used herein denote polypropylene and polyethylene, respectively, which have a high degree of short and long chain branching and a density range of about 0.91 to about 0.94 g/cc.

In the present invention, the at least one flexible polymeric fiber imparts flexibility and lubricity to the inventive composite yam, while the at least one wear-resistant polymeric fiber imparts strength and durability to the inventive composite yam. Depending on the intended use or performance requirement, the properties of the inventive composite yarn, such as flexibility and durability, may be controlled by fiber material selection and/or fiber strand arrangement. That is, the properties of the inventive composite yarn may be tuned by using different wear-resistant polymeric fiber and flexible polymeric fiber, varying the ratio of the wear-resistant polymeric fiber to the flexible polymeric fiber, and/or adjusting the orientation of the wear-resistant polymeric fiber and the flexible polymeric fiber within the inventive composite yam. In other words, various amounts of the at least one wear-resistant polymeric fiber and the at least one flexible polymeric fiber may be mixed and positioned to achieve a balance of flexibility and durability for the inventive composite yam. For example, the flexibility of the inventive yarn can be enhanced by reducing the ratio of the at least one wear-resistant polymeric fiber and the at least one flexible polymeric fiber; while the strength and durability of the inventive yarn can be enhanced by increasing the ratio of the at least one wear-resistant polymeric fiber and the at least one flexible polymeric fiber. When the at least one wear-resistant polymeric fiber is positioned to form a central core, and the at least one flexible polymeric fiber is positioned to form a periphery surrounding the central core, the resulting composite yarn possesses a flexible and lubricous surface and a strong core. Alternatively, when the at least one flexible polymeric fiber is positioned to form a central core, and the at least one wear-resistant polymeric fiber is positioned to form a periphery surrounding the central core, the resulting composite yarn possesses a strong and wear-resistant surface and a flexible core. When the flexibility and durability of the inventive composite yarn need to be evenly balanced, the at least one wear-resistant polymeric fiber and the at least one flexible polymeric fiber can be evenly blended in pairs. In addition, the polymeric fibers in the inventive composite yarn may be co-extruded filaments combining individual properties of various polymeric materials.

FIGS. 1A to 1D show the cross-section views of four embodiments of the inventive composite yarn that comprise multiple wear-resistant polymeric fibers and multiple flexible polymeric fibers. In FIG. 1A, the inventive composite yarn comprises 6 wear-resistant polymeric fibers and 11 flexible polymeric fibers in a bundle with the wear-resistant polymeric fibers positioned at the periphery of the bundle. In FIG. 1B, the inventive composite yarn comprises 12 wear-resistant polymeric fibers and 5 flexible polymeric fibers in a bundle wherein the flexible polymeric fibers are positioned to form a central core and the wear-resistant polymeric fibers are positioned to form a periphery surrounding the central core. In these drawings, light gray color, i.e., reference number 10, denotes a flexible polymeric fiber, and dark gray color, i.e., reference number 12, denotes a wear-resistant polymeric fiber. In FIG. 1C, the inventive composite yarn comprises 5 wear-resistant polymeric fibers and 12 flexible polymeric fibers in a bundle wherein the wear-resistant polymeric fibers are positioned to form a central core and the flexible polymeric fibers are positioned to form a periphery surrounding the central core. In FIG. 1D, the inventive composite yarn comprises 7 wear-resistant polymeric fibers and 7 flexible polymeric fibers in a bundle wherein the wear-resistant polymeric fibers and the flexible polymeric fibers are blended in pairs. That is, in FIG. 1D, the wear-resistant polymeric fibers and the flexible polymeric fibers are arranged in an even manner to form the inventive composite yarn.

The inventive composite yarn synergistically combines flexibility and durability, and thereby is particularly suitable to be used for the construction of graft materials. Unlike the prior art methods which blend yarns of different materials, the present invention blends various polymeric fibers to form a composite yarn. Comparing to a fabric prepared by blending different yarns to obtain specific characteristics, a fabric prepared from the inventive composite yarn can achieve the same or improved characteristics with reduced fabric thickness. Therefore, when used to construct graft materials for vascular grafts or other graft devices, the inventive composite yarn not only shows improved balance of desired properties, but also minimizes the overall material thickness needed to achieve the desired mechanical properties, thereby providing a thin-walled graft material with enhanced durability. Moreover, the inventive composite yarn provides a more homogeneous blend for graft materials, thus enhancing the performance of the graft material.

In one preferred embodiment of the present invention, the inventive composite yarn comprises polymeric fibers of polyethylene terphthalate and polymeric fibers of ultra high molecular weight polyethylene, wherein the total number of polymeric fibers are 20, and the ratio of polymeric fibers of polyethylene terphthalate and polymeric fibers of ultra high molecular weight polyethylene by number is about 1:4 to about 4:1.

The inventive composite yarn may be prepared through a spinning process, an air texturizing process, or other processes known to one skilled in the art. Methods of preparing composite yarn are well known in the art and detailed conditions for preparing the inventive composite yarn can be readily ascertained by one skilled in the art.

The inventive composite yarn may form graft materials utilizing any number of techniques, including weaving, knitting and braiding. Weaving involves the interlacing, at right angles, of two systems of threads known as warp and filling. Warp threads run lengthwise in a woven fabric and filling threads run cross-wise. Knitting is the process of making fabric by interlocking a series of loops of one or more threads. Braiding involves crossing diagonally and lengthwise several threads of any of the major textile fibers to obtain a certain width effect, pattern or style.

Depending on the intended use or performance requirement, the reinforced fiber graft material may in any shape or thickness. It is preferred that the fiber graft material is such a shape that it can be attached to an outside portion of one or more scaffold structures tightly. When the one or more scaffold structures comprise one or more stent segments, it is preferred that the reinforced fiber graft material is in a tubular shape. The wall thickness of the reinforced fiber graft material is determined primarily by weave density and yarn thickness or bulkiness. It is desirable to have the reinforced fiber graft material which is packed tight enough to prevent significant blood seepage, but not so tight that the yarn or fiber bundles pile up on each other. It is preferred that the reinforced fiber graft material has a wall thickness of 0.127 mm (0.005 inches) or less with a wall thickness of 0.076 mm (0.003 inches) or less more preferred.

## Claims

1. A composite yarn comprising at least one wear-resistant polymeric fiber (12) and at least one flexible polymeric fiber (10); wherein the at least one wear-resistant polymeric fiber (12) is more wear-resistant than the at least one flexible polymeric fiber (10) and the at least one flexible polymeric fiber (10) is more flexible than the at least one wear-resistant polymeric fiber (12) and wherein the ratio of the at least one wear-resistant polymeric fiber (12) to the at least one flexible polymeric fiber (10) by number is about 1:4 to about 4:1, and the total number of the at least one wear-resistant polymeric fiber (12) and the at least one flexible polymeric fiber (10) ranges from 5 to 150.

2. The composite yarn of claim 1, wherein the total number of the at least one wear-resistant polymeric fiber (12) and the at least one flexible polymeric fiber (10) ranges from 10 to 50.

3. The composite yarn of claim 1 has a denier ranging from about 15 to about 500.

4. The composite yarn of claim 1, wherein the at least one wear-resistant polymeric fiber (12) is positioned to form a central core, and the at least one flexible polymeric fiber (10) is positioned to form a periphery surrounding the central core.

5. The composite yarn of claim 1, wherein the at least one flexible polymeric fiber (10) is positioned to form a central core, and the at least one wear-resistant polymeric fiber (12) is positioned to form a periphery surrounding the central core.

6. The composite yarn of claim 1, wherein the at least one flexible polymeric fiber (10) and the at least one wear-resistant polymeric fiber (12) are blended in pairs.

7. The composite yarn of claim 1, wherein the at least one flexible polymeric fiber (10) is a fiber of polyamide, polyester, polyolefin, or fluorinated polymer.

8. The composite yarn of claim 1, wherein the at least one wear-resistant polymeric (12) fiber is a fiber of polyolefin, polyester, poly(ether amide), poly(ether ester), poly(ether urethane), poly(ester urethane), or poly(ethylene-styrene/butylene-styrene).

9. The composite yarn of claim 1, wherein the at least one flexible polymeric fiber (10) is a fiber of nylon 6, nylon 66, nylon 11, nylon 12, polyethylene terphthalate, polybutylene terephthalate, low density polypropylene, low density polyethylene, or poly(vinylidene fluoride).

10. The composite yarn of claim 1, wherein the at least one wear-resistant polymeric fiber (12) is a fiber of ultra high molecular weight polyethylene or ultra high molecular weight polypropylene.

## Patentansprüche

1. Verbundgarn, das wenigstens eine abriebfeste polymere Faser (12) und wenigstens eine flexible polymere Faser (10) umfasst; wobei die wenigstens eine abriebfeste polymere Faser (12) abriebfester ist als die wenigstens eine flexible polymere Faser (10) und die wenigstens eine flexible polymere Faser (10) flexibler ist als die wenigstens eine abriebfeste polymere Faser (12) und wobei das Verhältnis der wenigstens einen abriebfesten polymeren Faser (12) zu der wenigstens einen flexiblen polymeren Faser (10) nach Anzahl etwa 1:4 bis etwa 4:1 beträgt und die Gesamtanzahl der wenigstens einen abriebfesten polymeren Faser (12) und der wenigstens einen flexiblen polymeren Faser (10) von 5 bis 150 reicht.

2. Verbundgarn nach Anspruch 1 wobei die Gesamtanzahl der wenigstens einen abriebfesten polymeren Faser (12) und der wenigstens einen flexiblen polymeren Faser (10) von 10 bis 50 reicht.

3. Verbundgarn nach Anspruch 1, das einen Denier im Bereich von etwa 15 bis etwa 500 aufweist.

4. Verbundgarn nach Anspruch 1, wobei die wenigstens eine abriebfeste polymere Faser (12) so angeordnet ist, dass sie einen zentralen Kern bildet, und die wenigstens eine flexible polymere Faser (10) so angeordnet ist, dass sie eine Peripherie bildet, die den zentralen Kern umschließt.

5. Verbundgarn nach Anspruch 1, wobei die wenigstens eine flexible polymere Faser (10) so angeordnet ist, dass sie einen zentralen Kern bildet, und die wenigstens eine abriebfeste polymere Faser (12) so angeordnet ist, dass sie eine Peripherie bildet, die den zentralen Kern umschließt.

6. Verbundgarn nach Anspruch 1, wobei die wenigstens eine flexible polymere Faser (10) und die wenigstens eine abriebfeste polymere Faser (12) in Paaren gemischt sind.

7. Verbundgarn nach Anspruch 1, wobei die wenigstens eine flexible polymere Faser (10) eine Faser aus Polyamid, Polyester, Polyolefin oder fluoriertem Polymer ist.

8. Verbundgarn nach Anspruch 1, wobei die wenigstens eine abriebfeste polymere Faser (12) eine Faser aus Polyolefin, Polyester, Poly(ether-amid), Poly(ether-ester), Poly(ether-urethan), Poly(ester-urethan) oder Poly(ethylen-styrol/butylen-styrol) ist.

9. Verbundgarn nach Anspruch 1, wobei die wenigstens eine flexible polymere Faser (10) eine Faser aus Nylon 6, Nylon 66, Nylon 11, Nylon 12, Polyethylenterephthalat, Polybutylenterephthalat, Polypropylen mit niedriger Dichte, Polyethylen mit niedriger Dichte oder Poly(vinylidenfluorid) ist.

10. Verbundgarn nach Anspruch 1, wobei die wenigstens eine abriebfeste polymere Faser (12) eine Faser aus Polyethylen mit ultrahohem Molekulargewicht oder Polypropylen mit ultrahohem Molekulargewicht ist.

## Revendications

1. Fil composite comprenant au moins une fibre polymère résistante à l'usure (12) et au moins une fibre polymère flexible (10), dans lequel ladite fibre polymère résistante à l'usure (12) est plus résistante à l'usure que ladite fibre polymère flexible (10) et ladite fibre polymère flexible (10) est plus flexible que ladite fibre polymère résistante à l'usure (12) et dans lequel le rapport entre ladite fibre polymère résistante à l'usure (12) et ladite fibre polymère flexible (10) en nombre est d'environ 1:4 à environ 4:1, et le nombre total de fibres polymères résis-tantes à l'usure (12) et de fibres polymères flexibles (10) est compris entre 5 et 150.

2. Fil composite selon la revendication 1, dans lequel le nombre total de fibres polymères résis-tantes à l'usure (12) et de fibres polymères flexibles (10) est compris entre 10 et 50.

3. Fil composite selon la revendication 1, ayant un denier compris entre environ 15 et environ 500.

4. Fil composite selon la revendication 1, dans lequel ladite fibre polymère résistante à l'usure (12) est positionnée de façon à former un noyau central, et ladite fibre polymère flexible (10) est positionnée de façon à former une périphérie entourant le noyau central.

5. Fil composite selon la revendication 1, dans lequel ladite fibre polymère flexible (10) est positionnée de façon à former un noyau central, et ladite fibre polymère résistante à l'usure (12) est positionnée de façon à former une périphérie entourant le noyau central.

6. Fil composite selon la revendication 1, dans lequel ladite fibre polymère flexible (10) et ladite fibre polymère résistante à l'usure (12) sont mélangées par paires.

7. Fil composite selon la revendication 1, dans lequel ladite fibre polymère flexible (10) est une fibre de polyamide, polyester, polyoléfine ou polymère fluoré.

8. Fil composite selon la revendication 1, dans lequel ladite fibre polymère résistante à l'usure (12) est une fibre de polyoléfine, polyester, poly(éther amide), poly(éther ester), poly(éther uréthane), poly(ester uréthane), ou poly-(éthylène-styrène/butylène-styrène).

9. Fil composite selon la revendication 1, dans lequel ladite fibre polymère flexible (10) est une fibre de nylon 6, nylon 66, nylon 11, nylon 12, polyéthylène téréphtalate, polybutylène téréphtalate, polypropylène basse densité, poly-éthylène basse densité ou poly(vinylidène fluorure).

10. Fil composite selon la revendication 1, dans lequel ladite fibre polymère résistante à l'usure (12) est une fibre de polyéthylène à masse molé-culaire ultra-élevée ou un polypropylène à masse moléculaire ultra-élevée.
